(19) Europäisches Patentamt
European Patent Office
Office européen des brevets

(11) Veröffentlichungsnummer: **0 044 988**
**B1**

(12) # EUROPÄISCHE PATENTSCHRIFT

(45) Veröffentlichungstag der Patentschrift: **19.06.85**

(51) Int. Cl.⁴: **G 01 N 33/53**

(21) Anmeldenummer: **81105416.2**

(22) Anmeldetag: **11.07.81**

(54) **Trennung des freien und antikörpergebundenen Liganden für labelled-Ligand-Immunoassays.**

(30) Priorität: **30.07.80 DD 222959**

(43) Veröffentlichungstag der Anmeldung: **03.02.82 Patentblatt 82/5**

(45) Bekanntmachung des Hinweises auf die Patenterteilung: **19.06.85 Patentblatt 85/25**

(84) Benannte Vertragsstaaten: **CH DE FR GB LI SE**

(56) Entgegenhaltungen:
**WO - A - 81/02899**
**DE - A - 2 539 242**
**FR - A - 2 256 413**
**FR - A - 2 385 099**
**US - A - 4 048 298**
**US - A - 4 108 976**

**CLINICAL CHEMISTRY, Band 26, Nr. 4, April 1980, Seiten 666-667 Easton, Pennsylvania, U.S.A. H. HORN et al.: "Polyethylene glycol and second antibody in radioimmunoassay of human choriogonadotropin"**

(73) Patentinhaber: **VEB Arzneimittelwerk Dresden, Wilhelm-Pieck-Strasse 35, DDR-8122 Radebeul 1 (DD)**

(72) Erfinder: **Ziegler, Manfred, Dr. Dipl. Chem., Schulstrasse 15, DDR-2201 Karlsburg (DD)**
Erfinder: **Woltanski, Klaus-Peter, Dipl.-Chem., Schulstrasse 27, DDR-2201 Karlsburg (DD)**
Erfinder: **Diaz-Alonso, Jose, Manuel, Dipl.-Chem., Schulstrasse 32, DDR-2201 Karlsburg (DD)**

(74) Vertreter: **Puchberger, Rolf, Dipl. Ing. et al, Patentanwälte Dipl. Ing. Rolf Puchberger Dipl. Ing. Georg Puchberger Singerstrasse 13, A-1010 Wien (AT)**

**EP 0 044 988 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die Erfindung beinhaltet ein universelles Verfahren zur Trennung des freien und antikörpergebundenen markierten Antigens bzw. Haptens für alle Ligand-Immunoassay-Systeme zur quantitativen Bestimmung, vorwiegend biologisch aktiver Substanzen wie Hormone, Enzyme oder Pharmaka bis zu Konzentrationen um $10^{-12}$ mol/l, meistens in Körperflüssigkeiten. Das umfangreichste Anwendungsgebiet liegt beim Radioimmunoassay (RIA) und Enzymimmunoassay (EIA).

Nach Einführung des Radioimmunoassays (RIA) durch YALOW und BERSON (J. clin. Invest. 39 (1960) 1157) zur Insulinbestimmung im Serum wurden bis heute für über 200 Substanzen Ligand-Immunoassays publiziert. Sie beruhen auf der Messung der prozentualen Bindung des markierten Liganden $^{x}L$ durch den ligandspezifischen Antikörper $AK_1$ in Abhängigkeit von der zu bestimmenden Konzentration des unmarkierten Liganden L. Bei allen Methoden stellt sich für die ligandspezifische Immunreaktion folgendes Gleichgewicht ein:

markierter
Ligand ($^{x}L$)

$$+ \text{ Antikörper } (AK_1) \rightleftharpoons \underset{(B)}{^{x}L - AK_1} + \underset{(F)}{^{x}L}$$

Ligand (L)
unbekannte
Konzentration

Die Konzentration $^{x}L$ und $AK_1$ sind in einem Inkubationsansatz konstant gehalten. Nach Gleichgewichtseinstellung ist eine Trennung des freien (F) und antikörpergebundenen (B) markierten Liganden erforderlich. Da der Immunkomplex selbst löslich ist, wurden hierzu verschiedene Trennmethoden entwickelt, jedoch hat sich bisher keine universell vorteilhaft anwendbare Methode ergeben. Somit liegen die größten Unterschiede der publizierten Radioimmunoassays und kommerziell angebotenen RIA-Kits in der Durchführung der Trennung von B und F.

Die Trennung soll möglichst quantitativ erfolgen, hierdurch wird die untere Erfassungsgrenze eines RIA und auch seine Präzision wesentlich beeinflußt. Das Trennverfahren muß sich für einen hohen Probedurchsatz eignen. Das Gleichgewicht der ligandspezifischen Immunreaktion sollte durch die Trennung nicht nachweislich geändert werden. Da diese Anforderungen oft schwierig einzuhalten sind, wurden viele Trennmethoden entwickelt.

So wurden die Elektrophorese, Chromatographie und Ultrazentrifugation auf Grund verschiedener Mobilität von B und F zur Trennung angewendet. Als Nachteile für diese Methoden sind die starken Gleichgewichtsstörungen, der hohe Zeitaufwand und ein geringer Probendurchsatz zu nennen.

Eine Trennung kann auch erreicht werden, wenn B durch Äthanol, Dioxan, Polyäthylenglykol bei 125 g/l oder Ammoniumsulfat bei 215 g/l präzipitiert wird. Die beiden ersten, sonst eleganten Verfahren haben den Nachteil, daß mit zunehmenden Proteingehalt der markierte freie Ligand (F) zunehmend kopräzipitiert. Außerdem werden hierbei die Immunkomplexe niederaffiner Antikörper gespalten.

Eine andere Gruppe von Trennmethoden beruht auf der Adsorption des freien Liganden (F) an Aktivkohle, Ionenaustauscherharze, Cellulose oder Silicate. Der Nachteil dieser Methoden ist, daß auch B teilweise adsorbiert wird, wobei das Ausmaß der Bindung milieuabhängig ist.

Eine weitere Möglichkeit ist die chemische Fixierung des ligandspezifischen Antikörpers vor der Reaktion, z. B. an Cellulose oder Sepharose (solid-phase-method) bzw. an der Innenwand des Inkubationsröhrchens (tube-method), wobei eine gewöhnliche Zentrifugation bzw. ein einfaches Abgießen zur Trennung ausreicht. Diese Methoden haben die Nachteile, daß die immobilisierten Antikörper meistens in ihrer Affinität und damit die RIA in ihrer Empfindlichkeit vermindert sind. Der Verbrauch der meist teueren spezifischen Antikörper ($AK_1$) ist durch die Fixierung um ein Vielfaches erhöht. Außerdem müssen die Inkubationsröhrchen wegen der heterogenen Reaktionsphasen während der Inkubation ständig bewegt werden.

Alle bis hierher genannten Methoden sind physiko-chemischer Art. Eine weitere Gruppe von Trennverfahren sind unter dem Begriff der Doppelantikörpermethode zusammenzufassen. Hierbei erfolgt die Trennung von B und F dadurch, daß mittels eines zweiten Antikörpers ($AK_2$), der gegen das Immunglobulin der Spenderspezies für $AK_1$ gerichtet ist, der lösliche Immunkomplex $^{x}L$-$AK_1$ präzipitiert wird. Da der $AK_1$ in sehr niedriger Konzentration (Verdünnung des Antiserums 1 : $10^3$ bis 1 : $10^6$) eingesetzt wird, wird dem Inkubat ein Normalserum (NS) der Spezies des $AK_1$-Spenders bei einer Verdünnung von ca. 1 : 100 zugesetzt. Das Konzentrationsverhältnis von NS zu $AK_2$ ist so abgestimmt, daß eine maximale Immungpräzipitation erreicht wird. Nach Ablauf der Präzipitation wird B abzentrifugiert. Die Doppelantikörpertechnik ergibt den besten Trenneffekt zwischen B und F, die unspezifische Präzipitation (Bu) ist sehr niedrig. Der Nachteil dieser Methode ist, daß die Bindung des $^{x}L$-$AK_1$ an $AK_2$ durch Immunglobuline individueller Patienten (BRUNFELDT, K. und K. R. JÖRGENSEN, Acta Endocrin. 54 (1967) 347) kompetitiv gehemmt wird und somit falsch hohe Werte von L im Plasma bestimmt werden. Zudem wird die Präzipitation durch das Komplement quantitativ beeinflußt.

Um die genannten Nachteile zu vermeiden, wurde mit dem sogenannten Bindungsreagens, dem vorpräzipitierten spezifischen $AK_1$ mit $AK_2$ unter Zusatz von NS, gearbeitet (HALES, C. N. and P. J. RANDLE, Biochem. J. 88 (1963) 137). Hierbei blieb der Vorteil der geringen Bu erhalten, auch wird die Kreuzreaktion des Immunglobulins mit dem $AK_2$ und der Komplementeinfluß weitgehend unterbunden, aber gleichzeitig werden damit die Vorteile der homogenen Reaktionsphase für die ligandspezifische Immunreaktion aufgegeben.

Eine andere Variante wurde durch DEN HOLLANDER u. a. (J. of Immunol. Methods 1 (1972) 247) beschrieben. Hiernach wird $AK_2$ an einen festen Träger fixiert und der lösliche Immunkomplex $^{x}L\text{-}AK_1$ wird durch Immunadsorption vom freien Liganden $^{x}L$ getrennt. Da der fixierte $AK_2$ im Überschuß zugegeben werden kann, sind die Kreuzreaktionen des Immunglobulins mit $AK_2$ und die Komplementreaktion ohne Einfluß auf die quantitative Bindung des $^{x}L\text{-}AK_1$. Der Nachteil dieser Methode liegt darin begründet, daß bei der chemischen Trägerfixierung ein erheblicher Teil $AK_2$ inaktiviert wird, dadurch steigt der $AK_2$-Verbrauch. Zum anderen ist der Solidphase-$AK_2$ nicht stabil, man findet schon nach wenigen Tagen Aufbewahrung freie $AK_2$, wodurch dann falsch hohe Konzentration für den zu bestimmenden Liganden L gefunden werden, außerdem kann die unspezifische Bindung durch die Festphase erhöht sein. Hinzu kommt, daß die Trägerfixierung nach noch zum Teil unübersichtlichen nicht steuerbaren Reaktionen abläuft und somit die Kupplungsausbeute und der Inaktivierungsgrad für $AK_2$ und damit die Bindeeigenschaften des Solidphase-$AK_2$ für $AK_1$ sehr variieren können. Jedoch der größte Nachteil liegt in der Durchführung selbst, denn während der gesamten Inkubation (einige Stunden) zur $^{x}L\text{-}AK_1$-Bindung müssen die Proben geschüttelt werden.

Das Ziel der Erfindung ist die Einführung einer universellen Methode zur Trennung der freien (F) und antikörpergebundenen (B) Liganden in den vorteilhaft anzuwendenden Ligand-Immunoassay-Systemen zur Bestimmung z. B. von Hormonen, Enzymen, Pharmaka, spezifischen Proteinen u. a. Substanzen, vorwiegend in biologischen aber auch anderen Medien, unabhängig von der Markierungsart der Liganden (Radioimmunoassay, Enzymimmunoassay u. a. Immunoassays). Für den Erkenntnisgewinn in biochemischer und klinischer Forschung ist die hochspezifische, empfindliche Analytik eine wesentliche Voraussetzung, somit ist der Bedarf an den noch sehr teuren Testbestecken sehr hoch. Mit Einführung des erfindungsgemäßen universellen Trennverfahrens wäre eine Standardisierung in der Fertigung und Testdurchführung gegeben und damit gleichzeitig eine erhebliche Kosteneinsparung verbunden.

Die Aufgabe der Erfindung besteht darin, ein neues, universelles Trennverfahren zur Separation des freien (F) und antikörpergebundenen (B) Liganden für die Radio-, Enzym-, Spin- oder allgemein labelled-Ligand-Immunoassays einzuführen.

Erfindungsgemäß wird vorgeschlagen, daß die Trennung mit Hilfe eines immuno-immobilisierten Präzipitationsreagens (IIPR), bestehend aus einem feindispersen Immunpräzipitat, das nach Kurzzeitinkubation von Normalserum [NS) der Spezies der Spendertiere der ligandspezifischen Antiseren mit einem Antiserum ($AK_2$) gegen die Immunglobline dieser Spezies bei optimiertem Antigen/Antikörper-Verhältnis erhalten wurde, vorgenommen wird.

Das neue Trennverfahren zeichnet sich dadurch aus, daß die Doppelantikörpermethode mit ihren Vorteilen in der Durchführung so modifiziert wurde, daß zusätzlich die Vorzüge der Solidphasenmethode ausgenutzt werden, bei gleichzeitiger Beibehaltung der homogenen Reaktionsphase für die ligandspezifische Immunreaktion, ohne die chemische Fixierung des, den ligandspezifischen Antikörper ($AK_1$) bindenden, zweiten Antikörpers ($AK_2$) und die damit verbundene teilweise Inaktivierung des $AK_2$.

Die Kombination der genannten Vorteile der verschiedenen Trennmethoden ist dadurch gelungen, daß nach Ablauf der ligandspezifischen Immunreaktion in homogener, flüssiger Phase, der lösliche Immunkomplex $^{x}L\text{-}AK_1$ durch Zugabe eines feindispersen Immunpräzipitats IIPR (immuno-immobilisiertes Präzipitationsreagens), das durch Immunpräzipitation des $AK_2$ mit einem NS der Spezies des $AK_1$-Spenders zubereitet wurde, gebunden wird:

$$^{x}L - AK_1\ (B) + {}^{x}L\ (F) + IIPR \rightleftharpoons {}^{x}L - AK_1 - IIPR \downarrow (B) + {}^{x}L \uparrow (F)$$

Nach einer Inkubation von nur 30 min mit dem IIPR wird jede Probe mit 2 ml des ammoniumsulfathaltigen Assay-Puffers vermischt, wobei die Endkonzentration des $(NH_4)_2SO_4$ 1,16 mol/l betragen soll. Nach weiteren 30 min werden die Proben 15 min bei 2000 g zentrifugiert. Ein zusätzlicher Waschschritt ist in der Regel nicht erforderlich. Anstelle des $(NH_4)_2SO_4$ kann auch Polyäthylenglykol 6000 (Merck, Darmstadt) mit der Endkonzentration von 30 g/l verwendet werden.

Das IIPER wird zweckmäßig im Großansatz (mehrere Tausend Ampullen) hergestellt. Das optimale Konzentrationsverhältnis des $AK_1$-speziesgleichen Normalserums (NS) und des zweiten Antiserums ($AK_2$), das gegen $AK_1$-speziesgleiches Immunglobulin gerichtet ist, wird vorher ausgetestet.

Hierzu werden z. B.

0,1 ml NS-Verdünnung (1 : 400) mit
0,1 ml der $AK_2$-Verdünnungsreihe (1 : 2 bis 1 : 1000) 1 h bei Raumtemperatur (RT) inkubiert.
Anschließend werden

0,2 ml eines ${}^{x}$L-$AK_1$-Inkubates zugegeben, nach
30 min Inkubation bei RT wird das Inkubat mit
2,0 ml 1,4 mol/l $(NH_4)_2SO_4$-Assaypuffer vermischt und nach weiteren
30 min bei 2000 g für 15 min zentrifugiert.

Der Überstand wird verworfen, die Aktivität im Präzipitat gemessen. Aus der $AK_2$-Verdünnung, die sicher die maximale Bindung des ${}^{x}$L-$AK_2$ garantiert, wird das optimale NS/$AK_2$ Verhältnis für den Großansatz zur Herstellung des IIPR ermittelt.

Um gleichbleibend gute Binde- und Präzipitationseigenschaften des IIPR zu garantieren, werden die beiden Komponenten erfindungsgemäß so in einer Ampulle abgefüllt und lyophilisiert, daß die Reaktion miteinander bis zur Anwendung des IIPR ausgeschlossen bleibt: Pro Ampulle werden ca. 0,5 ml $AK_2$ tiefgefroren, danach werden in jede Ampulle 0,05 ml der NS-Verdünnung pipettiert. Das NS gefriert sofort, ohne daß das $AK_2$ auftaut. Anschließend wird lyophilisiert.

Aus laborpraktischen Gründen werden das $AK_2$ und NS bei Einhaltung des optimalen Konzentrationsverhältnisses so dosiert, daß jede Ampulle IIPR für 100 Teströhrchen enthält. Zur Anwendung des IIPR wird das Lyophilisat einer Ampulle mit 10 ml Assay-Puffer gelöst, nach 1 h Vorinkubation bei RT werden 10 µl des IIPR pro Teströhrchen pipettiert.

Da fast ausschließlich die ligandspezifischen Antiseren durch Immunisierung von Meerschweinchen (GP) und Kaninchen (Rab) gewonnen werden, ist eine Herstellung von nur zwei IIPR (GP-IIPR und Rab-IIPR) zur B/F-Trennung bei den genannten Immunoassays erforderlich. Somit führt die Standardisierung der B/F-Trennung nach der erfindungsgemäßen Methode zu einer hohen Effektivität bei der Produktion der Teststrecke, zusätzlich kommt es zur Vereinfachung der Durchführung der RIA, EIA und anderer Immunoassay für den Anwender, z. T. sind sehr unterschiedliche Laborausrüstungen z. B. für die verschiedenen Radioimmunoassays erforderlich.

Die folgenden Beispiele sollen die prinzipielle Anwendungsmöglichkeit des beschriebenen Trennverfahrens mit Hilfe des IIPR illustrieren.

## Beispiel 1

### Radioimmunoassay für Pankreasglukagon im Plasma

0,5 ml der Plasmaproben werden mit
0,1 ml eines verdünnten Kaninchen-anti-Glukagon-Serums 2 Tage bei 4° C und nach Zusatz von
0,05 ml 125J-Glukagonlösung
20 h bei 4° C inkubiert.
Danach werden zur R/F-Trennung
0,1 ml Rab-IIPR zugegeben und
30 min bei RT inkubiert, mit
2,0 ml 1,6 mol/l $(NH_4)_2SO_4$ im Assay-Puffer gemischt und nochmals
30 min bei RT inkubiert.
Nach anschließender Zentrifugation für 15 min bei 2000 g wird der Überstand mit dem freien 125J-Glukagon (F) abgegossen und die Radioaktivität im Präzipitat gemessen.

Der so durchgeführte RIA für Pankreasglukagon zeigt bei einer unspezifischen Bindung des 125J-Glukagons von 3,4 – 0,4% und einem Präzipitationsindex von 3,4 ± 0,3% ($\bar{x}$ ± SEM, n = 14) durch 100 pg Glukagon im Inkubat einen Abfall des gebundenen 125J-Glukagons (B) auf 38% gegenüber B bei Abwesenheit von Glukagon.

## Beispiel 2

### Radioimmunoassay für Insulin im Plasma

0,05 ml der Plasmaproben werden mit
0,1 ml eines verdünnten Meerschweinchen-anti-Insulin-Serums 20 h bei RT und nach Zusatz von
0,1 ml 125J-Insulin
90 min bei RT inkubiert.
Danach werden zur B/F-Trennung
0,1 ml GP-IIPR zugegeben und
30 min bei RT inkubiert, mit
2,0 ml 1,36 mol/l $(NH_4)_2SO_4$ im Assay-Puffer gemischt und nochmals
30 min bei RT inkubiert.
Nach anschließender Zentrifugation für 15 min bei 2000 g wird der Überstand mit dem freien 125J-Insulin (F) abgegossen und die Radioaktivität (B) im Präzipitat gemessen.

Der so durchgeführte RIA für Insulin zeigt bei einer unspezifischen Bindung des 125J-Insulins von 2,45 ± 0,05% und einem Präzipitationsindex von 2,6 ± 0,3% ($\bar{x}$ ± SEM, n = 7) durch 300 pg (0,3 µE) Insulin im Inkubat, einen Abfall des gebundenen 125J-Insulins (B) auf 12,6% gegenüber von B bei Abwesenheit von unmarkiertem Insulin.

**Patentansprüche**

1. Verfahren zur Trennung des freien (F) und antikörpergebundenen (B) Liganden bei der Durchführung der labelled-Ligand-Immunoassays, dadurch gekennzeichnet, daß die Trennung mit Hilfe eines immuno-immobilisierten Präzipitationsreagens (IIPR), bestehend aus einem feindispersen Immunpräzipitat, das nach Kurzzeitinkubation von Normalserum (NS) der Spezies der Spendertiere der ligandspezifischen Antiseren mit einem Antiserum ($AK_2$) gegen die Immunglobuline dieser Spezies bei optimiertem Antigen/Antikörper-Verhältnis erhalten wurde, vorgenommen wird.

2. Verfahren nach Anspruch 1, dadurch gekennzeichnet, daß das immuno-immobilisierte Präzipitationsreagens (IIPR) erst nach Ablauf der ligandspezifischen Immunreaktion in homogener Phase mit dem dabei erzeugten spezifischen Inkubat vermischt wird und die B/F-Trennung anschließend durch Zentrifugation und Abgießen des Überstandes erreicht wird.

3. Verfahren nach den Ansprüchen 1 und 2, dadurch gekennzeichnet, daß das feindisperse immuno-immobilisierte Präzipitationsreagens (IIPR) kurz vor seiner Anwendung durch Aufnahme der lyophilisierten Komponenten Normalserum (NS) und Antiserum ($AK_2$), die durch Anwendung eines Gefrierzwischenschrittes unvermischt in einer Ampulle gelagert werden, mit Puffer erzeugt wird.

4. Verfahren nach den Ansprüchen 1 bis 3, dadurch gekennzeichnet, daß durch Zusatz von Ammoniumsulfat oder Polyäthylenglykol (PEG) der zeitliche Aufwand für die B/F-Trennung auf nur 1 h reduziert wird, wobei die Konzentration der genannten Zusätze und die Präzipitationsdauer so gewählt werden, daß die ohne $(NH_4)_2SO_4$ — bzw. PEG-Zusatz löslichen $^{x}L$-$AK_1$-$AK_2$-Komplexe aber nicht der markierte freie (P) Ligand ($^{x}L$) und die Immunglobuline selbst präzipitieren.

5. Verfahren nach den Ansprüchen 1 bis 4, dadurch gekennzeichnet, daß der zu bestimmende Ligand jede Substanz sein kann, gegen die direkt oder nach Konjugation an einem Trägermaterial spezifische Antikörper erzeugt werden können und die im Ligand-Immunoassay als Antigen oder Hapten reagiert, wobei die Bestimmung von Liganden im Plasma der Spezies des Spendertieres für $AK_1$ ausgeschlossen ist.

6. Verfahren nach den Ansprüchen 1 bis 5 dadurch gekennzeichnet, daß für den Ligand-Immunoassay der Ligand selbst oder eine antigene bzw. haptene ligandspezifische Struktureinheit mit einem empfindlich zu messenden Marker, vorzugsweise einem radioaktiven Isotop oder einem Enzym, bei Erhaltung der Reaktivität zum spezifischen Antikörper, etikettiert, eingesetzt wird.

**Claims**

1. Method for the separation of the free (F) and antibody-bound (B) ligand in the performance of the labelled-ligand immuno-assays, characterized in that the separation is carried out by way of an immuno-immobilized precipitation reagent (IIPR) consisting of a finely dispersed immuno precipitate which has been gained after short time-incubation of normal serum (NS) of the donating animals of ligand specific antisera with an antiserum ($AK_2$) against the immuno globulines of this species at optimized antigen-antibody-ratio.

2. Method according to claim 1, characterized in that the immuno-immobilized precipitation reagent (IIPR) is not admixed but after completion of the ligand specific immuno reaction in homogenous phase with specific incubate produced thereby and the B-F-separation is gained then by zentrifugation and decanting the supernatant

3. Method according to claims 1 and 2, characterized in that the finely dispersed, immuno-immobilized precipitation reagent (IIPR) is produced with buffer shortly prior to its utilisation by inclusion of the lyophilized components normal serum (NS) and antiserum ($AK_2$) being stored unmixed in an ampoule using an intermediary freezing step.

4. Method according to the claims 1 to 3, characterized in that by addition of ammonium sulfate or poly ethylen glycol (PEG] the time consumption for the B-F-separation is reduced just to 1 hour, the concentration of the said additions and the precipitation time being choosen that way that the $^{x}L$-$AK_1$-$AK_2$-complexes being solveable without $(NH_4)_2SO_4$ — or PEG-addition, but not the marked free (F) ligand($^{x}L$) and the immuno globulines are precipitating themselves.

5. Method according to the claims 1 to 4, characterized in that the ligand to be determined might be any substance against which directly or after conjugation at a carrier material, specific antibodies can be produced and which reacts in the ligand-immuno-assay as antigene or haptene, the determination of ligands in the plasma of the species of the donating animal for $AK_1$ being excluded.

6. Method according to claims 1 to 5, characterized in that for the ligand-immuno-assay the ligand itself or an antigeneous or hapteneous ligand specific structure unit labelled with a marker to be

sensibly detected is used, preferably with a radioactive isotope or an enzyme in maintaining the reactivity to the specific antibody.

## Revendications

1. Procédé de séparation du ligand libre (F) et du ligand lié aux anticorps (B) dans la mise en œuvre d'un immuno-essai à ligand marqué, caractérisé par le fait que la séparation est effectuée à l'aide d'un réactif de précipitation immuno-immobilisé (IIPR), constitué par un immuno-précipité finement dispersé qui a été obtenu après incubation de courte durée de sérum normal (NS) de l'espèce d'animaux donneurs des antisérums spécifiques des ligands avec un antisérum ($AK_2$) contre les immuno-globulines de cette espèce avec un rapport antigène/anticorps optimisé.

2. Procédé selon la revendication 1, caractérisé par le fait que le réactif de précipitation immuno-immobilisé (IIPR) est mélangé seulement après la fin de l'immuno-réaction spécifique du ligand en phase homogène avec l'incubat spécifique alors produit et que la séparation B/F est atteinte ensuite par centrifugation et décantation du surnageant.

3. Procédé selon les revendications 1 et 2, caractérisé par le fait que le réactif de précipitation immuno-immobilisé (IIPR) finement dispersé est produit avec du tampon par réception, juste avant sa mise en œuvre, du composant lyophilisé de sérum normal (NS) et de l'antisérum ($AK_2$) qui sont conservés non mélangés dans une ampoule par mise en œuvre d'une étape intermédiaire de congélation.

4. Procédé selon les revendications 1 à 3, caractérisé par le fait que par addition de sulfate d'ammonium ou de polyéthylèneglycol (PEG) on réduit la durée pour la séparation B/F à seulement 1 heure, la concentration des additifs indiqués et la durée de précipitation étant choisis de façon telle que les complexes $^{x}L$-$AK_1$-$AK_2$ solubles sans addition de $(NH_4)_2SO_4$ ou de PEG mais non pas le ligand ($^{x}L$) marqué libre (F) et les immuno-globulines eux-nêmes précipitent.

5. Procédé selon les revendications 1 à 4, caractérisé par le fait que le ligand à doser peut être constitué par toute substance contre laquelle il est possible de produire directement ou après conjugaison sur un matériau de support des anticorps spécifiques et qui réagissent dans le ligand-immuno-essai comme antigène ou haptène, le dosage des ligands dans le plasma le l'espèce de l'animal donneur étant exclu pour l'$AK_1$.

6. Procédé selon les revendications 1 à 5, caractérisé par le fait que pour le ligand-immuno-essai le ligand lui-néme ou une unité structurelle antigène ou hapténe spécifique du ligand est mis en œuvre, marqué par un marqueur à mesurer de façon sensible, de préférence un isotope radio-actif ou une enzyme, la réactivité à l'égard de l'anticorps spécifique étant maintenue.